# EUROPEAN PATENT APPLICATION

(11) **EP 4 474 390 A1**
(43) Date of publication of application: **11.12.2024**
(21) Application number: 23382540.5
(22) Date of filing: 05.06.2023
(51) Int. Cl.: C07K 14/47, C12N 9/00, C07K 7/08, G01N 33/566

(54) **HIGH AFFINITY SUMO TRAPS**

(71) Applicant: Asociación Centro de Investigación Cooperativa en Biociencias - CIC bioGUNE, 48160 Derio (ES)
(72) Inventor: SUTHERLAND, James D., E-48160 Derio, Vizcaya (ES); BARRIO OLANO, María Rosa, E-48160 Derio, Vizcaya (ES)
(74) Representative: ABG Intellectual Property Law, S.L.

(57) **Abstract**

The present invention relates to methods for the purification by affinity binding of SUMO (Small-ubiquitin modifier) and SUMO-conjugate proteins by the use of fusion proteins comprising tandem arrangements of high affinity SUMO interacting motifs.

## Description

### TECHNICAL FIELD OF THE INVENTION

The present invention relates to the field of purification of target proteins based on their affinity towards specific binding reagents. In particular, the invention relates to methods for the purification by affinity binding of SUMO (Small-ubiquitin modifier) and SUMO-conjugate proteins by the use of fusion proteins comprising tandem arrangements of SUMO interacting motifs.

### BACKGROUND OF THE INVENTION

Post-translational modifications of proteins is an essential process in essentially all biological processes. These post-translational modifications include modification by small chemical groups, including phosphorylation, methylation, and acetylation, and modification by small proteins belonging to the ubiquitin family. SUMO (Small Ubiquitin MOdifier) is an ubiquitin-like protein involved in post- translational modification of critical cellular factors controlling protein localisation, transcription, DNA repair or cell cycle progression among other functions. Protein SUMOylation is defined as the covalent modification of target proteins with SUMO molecules on the lysine of the canonical consensus sequence ψK×E (where ψ is a large hydrophobic aminoacid and x any aminoacid). Modification of proteins by the small ubiquitin modifier (SUMO) results in very diverse outcomes. The first identified targets of SUMO, the oncogenic protein PML (promyelocytic leukemia) and the nucleocytoplasmatic transport protein RanGAPI (RanGTPase-activating I), allowed to connect SUMO functions to nuclear localization. The identification and characterization of hundreds of SUMO substrates suggest the role of SUMO as regulator of protein activity and stability.

SUMO proteins only have a 20% of identity with ubiquitin, however, have a similar three- dimensional structure. Three forms of SUMO (SUMO-1, SUMO-2 and SUMO-3) are ubiquitously expressed and migrate with an apparent molecular weight of around lOkDa in a denaturing polyacrylamide gel. SUMO-1 shares only 50% of identity with SUMO-2 and SUMO-3, but SUMO-2 and SUMO-3 are 97% identical, and form a distinct subfamily known as SUMO 2/3.

The consequences of the SUMOylation of a new protein target are difficult to predict as the changes of conformation, creation or masking interaction surfaces may affect activity, stability and localization of modified proteins. SUMOylated proteins are recognized by SUMO interacting motifs (SIMs) present in a large diversity of proteins includingpromyelocyte leukemia (PML), death domain-associated protein (Daxx), ubiquitin-like modifier activating enzyme 2 (UBA2), protein inhibitors of activated STAT (PIAS) and RING finger protein 4 (RNF4) ligases. SIMs are often present within SUMO substrates or enzymes regulating the levels of SUMOylated proteins. SIMs are present in many key cellular mediators and therefore the identification of new proteins containing SIMs can be important to further understand SUMO-dependent regulatory mechanisms.

The affinity of SIM and SUMO usually is low, around the high micromolar range, which is normally due to the reduced surface of interaction. To increase such affinity, proteins usually possess more than one SIM. In order to study the mechanisms of post-translation modification, new tools which improve the affinity of SUMO proteins for SUMOylation targets are required.

### SUMMARY OF THE INVENTION

The inventors of the present invention have generated protein constructs containing several small ubiquitin-related modifier (SUMO) interacting motif (SIM) arranged in tandem as to improve the detection and purification of sumoylated proteins.

Therefore a first aspect of the present invention relates to a polypeptide comprising at least 2 motifs, wherein each motif comprises a small ubiquitin-related modifier (SUMO) interacting motif (SIM), wherein the SIM comprises the sequence according to SEQ ID NO: 1 or a functionally equivalent variant thereof.

Another aspect of the present invention relates to a polynucleotide encoding the polypeptide according to the invention.

One more aspect of the present invention relates to a vector comprising the polynucleotide according to the invention.

A further aspect relates to a host cell comprising the vector according to the invention.

Yet another aspect of the present invention relates to the use of the polypeptide according to the invention, for the identification and/or isolation of SUMO substrates.

Another aspect of the present invention relates to an *in vitro*/*ex vivo* method for the isolation of SUMO substrates from a sample comprising the steps of:
i) incubating the polypeptide according to the invention with the sample in conditions allowing for said polypeptide to interact with at least one substrate present in said sample; and
ii) recovering any SUMO substrates which is bound to the polypeptide.

A further aspect of the present invention relates to an *in vitro*/*ex vivo* method for the identification of SUMO substrates from a sample comprising the steps of:
i) incubating the polypeptide according to the invention with the sample in conditions allowing for said polypeptide to interact with at least one substrate of the proteins present in said sample;
ii) recovering any SUMO substrates which is bound to the polypeptide; and
iii) identifying any of the SUMO substrates recovered in the previous step.

One more aspect relates to an *in vitro*/*ex vivo* method for the detection of SUMO substrates in a sample comprising the steps of:
i) incubating the polypeptide according to the invention with the sample in conditions allowing for said polypeptide to interact with at least one substrate of the proteins present in said sample;
ii) detecting the polypeptide of the previous step in the sample, wherein the detection of the polypeptide indicates the presence of any SUMO substrates.

A final aspect of the present invention relates to a kit comprising the polypeptide according to the invention and/or the polynucleotide according to the invention and/or the vector according to the invention and/or the host cell according to the invention.

### BRIEF DESCRIPTION OF THE FIGURES

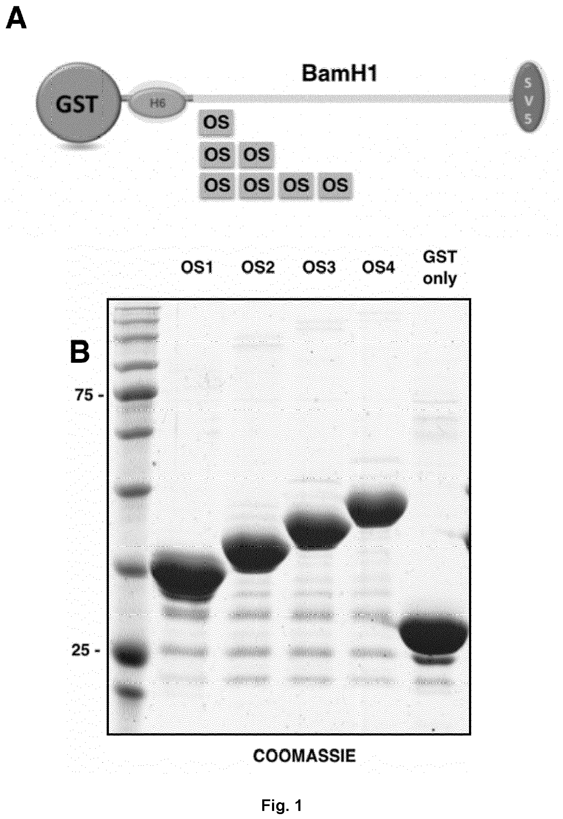

**Figure 1****. High affinity SUMO trap design and recombinant expression. A.** Schematic diagram of the molecular traps based on optimal SUMO2-binding SIM motifs (OptiSIM, SEQ ID NO: 3). The traps were designed as fusions with GST-HIS tags at the N-terminus and V5-epitope tag at the C-terminus. This is the same backbone as SUBE-L SUMO traps to allow direct comparison. B. OptiSIM-based molecular traps express well as recombinant proteins in bacteria and possess the correct molecular weights, depending on the number of OS repeats. With single-step glutathione-sepharose purification, the observed purity is >95%.

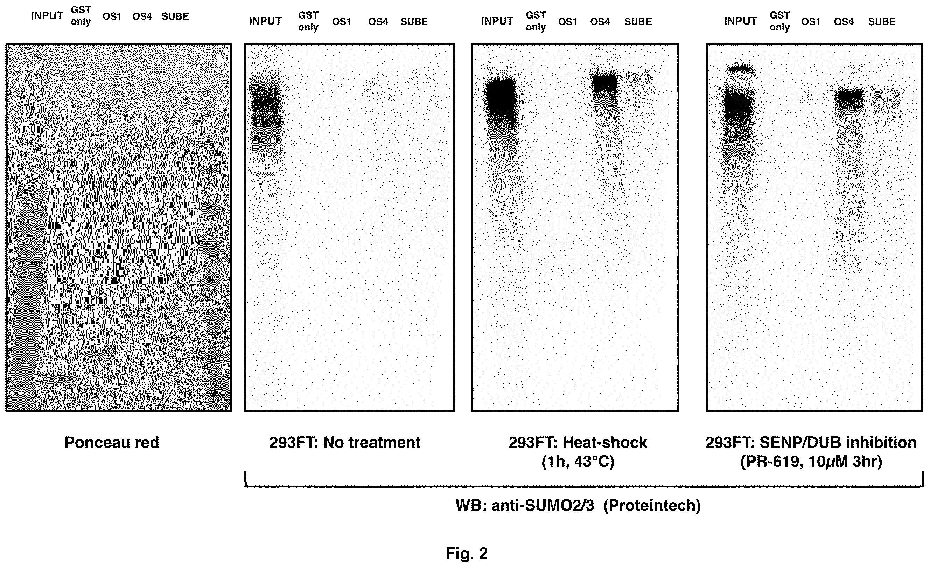

**Figure 2****. OS4 trap improves capture of polySUMOylated proteins.** A pulldown experiment is shown, comparing the ability of different molecular traps to capture polySUMOylated conjugates. The novel trap (OS4) is compared with either a single SIM version (OS1) or the previously described RNF4-SIM-based trap, SUBE-L. Different extracts are shown, with baseline versus induced levels of polySUMOylation (no treatment versus heat shock, PR-619 treated, respectively). SUMO2/3 antibody detects an enrichment in the capture of polySUMOylated conjugates when using OS4, especially in extracts with induced polySUMOylation.

### DETAILED DESCRIPTION OF THE INVENTION

The inventors of the present invention have generated protein constructs containing several small ubiquitin-related modifier (SUMO) interacting motif (SIM) arranged in tandem as to improve the detection and purification of sumoylated proteins.

### Polypeptides of the invention

Therefore, a first aspect of the present invention relates to a polypeptide comprising at least 2 motifs, wherein each motif comprises a small ubiquitin-related modifier (SUMO) interacting motif (SIM), wherein the SIM comprises the sequence according to SEQ ID NO: 1 or a functionally equivalent variant thereof.
SEQ ID NO: 1
EGDEDDDIIVVDDDDDEGE

The term "polypeptide" as used herein refers to a linear chain of amino acid residues of any length, joined together with peptide bonds. The term "peptide" as used herein, refers to a linear chain of amino acids as a polypeptide, although shorter than that of a polypeptide. It generally refers to amino acid chains of 2-50 amino acids. It will be understood that the terms "peptide bond", "peptide", "polypeptide" and "protein" are known to the person skilled in the art.

The term "motif" as used herein, refers to the pattern of unmodified and modified amino acids present in the protein of the invention.

The term "small ubiquitin-related modifier", or its acronym "SUMO", as used herein, refers to a family of small proteins that are covalently attached to and detached from other proteins in cells to modify their function. This process is called SUMOylation (also written sumoylation). SUMOylation is a post-translational modification involved in various cellular processes, such as nuclear-cytosolic transport, transcriptional regulation, apoptosis, protein stability, response to stress, and progression through the cell cycle.

SUMO proteins are similar to ubiquitin and are considered members of the ubiquitin-like protein family. SUMOylation is directed by an enzymatic cascade analogous to that involved in ubiquitination. In contrast to ubiquitin, SUMO is not used to tag proteins for degradation.

The term "SUMO-interacting motif", or its acronym "SIM", as used herein refers to the target site of proteins which interact with SUMO members through non-covalent interactions between the target protein and the SUMO protein. The number of SIMs in the target protein increases the probability of an interaction between the target protein and the SUMO. Therefore, in a particular embodiment of the polypeptide of the invention the polypeptide comprises between 2 and 7 motifs. In another particular embodiment the polypeptide of the invention comprises 3, 4, 5 or 6 motifs, preferably 4 motifs.

Not only is the number of SIMs of importance to increase the probability of an interaction between the target protein and the SUMO, the arrangement of said SIMs also affects said interaction probability. In a particular embodiment of the polypeptide of the invention, the motifs are arranged in tandem. The term "tandem" as used herein refers to the presence of at least two motifs adjacent to each other in the polypeptide of the invention. The expression "adjacent to each other" is understood to refer equally to motifs which comprise only the SIM and are contiguous to each other, without any amino acids flanking the SIMS, or motifs which are connected via short peptide sequences such as linkers, preferably wherein said short peptide sequences have less than 6 amino acids in length.

In order to be adjacent to each other and maintain optimal function, flexible linkers may be used as to provide improved function in terms of affinity, orientation and functionality of SIM-SUMO associations. In a particular embodiment of the polypeptide of the invention the motif further comprises flexible linkers flanking the SIMs. The term "flexible linker or spacer" as used herein, refers to a sequence hinge region between SUMO interacting motifs, allowing them to move independently of one another while maintaining the three-dimensional shape of the individual domains. In that sense, a flexible sequence linker according to the invention would be a hinge region characterized by a structural softness that enables this motion. In a more particular embodiment of the polypeptide of the invention the flexible linker comprises at least 2 amino acids selected from the group consisting of glycine, serine, alanine and threonine. In a further polypeptide of the invention the flexible linkers are glycine-serine flexible linkers and comprise a sequence consisting of GS or the sequence according to SEQ ID NO: 2.
SEQ ID NO: 2
GGSGG

The term "flanking" as used herein means that the flexible linker is present in both the N-terminus and in the C-terminus regions of the motif.

In a particular embodiment of the polypeptide of the invention the motif comprises the sequence according to SEQ ID NO: 3 or a functionally equivalent thereof. In a more particular embodiment of the polypeptide of the invention the motif consists of the sequence according to SEQ ID NO: 3.
SEQ ID NO: 3
GSEGDEDDDIIWDDDDDEGEGGSGG

The term "functionally equivalent thereof" as used herein refers to a SIM peptide as present in the polypeptide according to the invention that preferably retains at least one biological function or activity of the specific amino acid sequence of a SUMO interacting motif, preferably the ability to bind to SUMO or to a SUMOylated protein. The function of SIM peptide, in particular its ability to bind to SUMO or to a SUMOylated protein, can be determined by assays known by the skilled person, such as disclosed in Tanaka et al. (2010, Biosci Biotechnol Biochem; 74(6):1302-5) and Husnjak et al., (Methods Mol Biol. 2016;1475:79-98). In a particular embodiment, the functionally equivalent variant of the SIM preserves at least 50%, at least 60%, at least 70%, at least 80%, at least 90%, at least 95%, at least 98%, the 100% or higher of the ability of SIM of SEQ ID NO: 1 to bind to SUMO or to a SUMOylated protein.

Therefore in a particular embodiment of the polypeptide of the invention, the SIM functionally equivalent variant comprises or consists of a sequence with at least 80%, at least 85%, at least 90%, at least 95%, at least 96%, at least 97%, at least 98%, at least 99%, at least 99.9% identity with SEQ ID NO: 1. In another particular embodiment of the polypeptide of the invention, the SIM consist of the sequence according to SEQ ID NO: 1.

The terms "identity", "identical" or "percent identity" in the context of two or more amino acid or nucleotide sequences, refer to two or more sequences or subsequences that are the same or have a specified percentage of nucleotide residues that are the same, when compared and aligned (introducing gaps, if necessary) for maximum correspondence, not considering any conservative substitutions as part of the sequence identity. The percent identity can be measured using sequence comparison software or algorithms or by visual inspection. Various algorithms and software are known in the art that can be used to obtain alignments of amino acid or nucleotide sequences. Publicly available software programs can be used to align sequences. Appropriate parameters for maximal alignment by particular alignment software can be determined by one skilled in the art. In certain embodiments, the default parameters of the alignment software are used. In certain embodiments, the percentage identity "X" of a first nucleotide sequence to a second nucleotide sequence is calculated as 100 x (Y/Z), where Y is the number of nucleotide residues scored as identical matches in the alignment of the first and second sequences (as aligned by visual inspection or a particular sequence alignment program) and Z is the total number of residues in the second sequence. If the second sequence is longer than the first sequence, then the global alignment taken the entirety of both sequences into consideration is used, therefore all letters and null in each sequence must be aligned. In this case, the same formula as above can be used but using as Z value the length of the region wherein the first and second sequence overlaps, said region having a length, which is substantially the same as the length of the first sequence.

For instance, 95% identical to a reference sequence according to the present invention, the parameters are set such that the percentage of identity is calculated over the full length of the reference nucleotide sequence and that gaps in homology of up to 5% of the total number of nucleotides in the reference sequence are allowed.

SIMs generally consist of a four-residue-long hydrophobic stretch of amino acids with aliphatic non-polar side chains flanked on one side by negatively charged amino acid residues.

Therefore in a particular embodiment of the polypeptide of the invention the SIM functionally equivalent variant comprises or consists of the amino acid sequence of SEQ ID NO: 1 where:
- the amino acid at position 8 and/or position 9 of SEQ ID NO: 1 is replaced by an amino acid selected from leucine (L),valine (V), methionine (M) or phenylalanine (F); and/or
- the amino acid at position 10 and/or 11 of SEQ ID NO: 1 is replaced by an amino acid selected from isoleucine (I), leucine (L), methionine (M) or phenylalanine (F); and/or
- the amino acid at position 1, 4, 17, and/or 19 of SEQ ID NO: 1 is replaced by the amino acid aspartic acid (D); and/or
- the amino acid at position 3, 5, 6, 7, 12, 13, 14, 15, and/or 16 of SEQ ID NO: 1 is replaced by the amino acid glutamic acid (E); and/or
- the amino acid at position 2 and/or 18 of SEQ ID NO: 1 is replaced by the amino acid alanine (A) or serine (S).

In particular embodiment, the SIM functionally equivalent variant comprises or consists of the amino acid sequence of SEQ ID NO: 1 where the amino acid at position 8 and/or position 9 of SEQ ID NO: 1 is replaced by an amino acid selected from leucine (L),valine (V), methionine (M) or phenylalanine (F).

In particular embodiment, the SIM functionally equivalent variant comprises or consists of the amino acid sequence of SEQ ID NO: 1 where the amino acid at position 10 and/or 11 of SEQ ID NO: 1 is replaced by an amino acid selected from isoleucine (I), leucine (L), methionine (M) or phenylalanine (F).

In particular embodiment, the SIM functionally equivalent variant comprises or consists of the amino acid sequence of SEQ ID NO: 1 where the amino acid at position 1, 4, 17, and/or 19 of SEQ ID NO: 1 is replaced by the amino acid aspartic acid (D).

In particular embodiment, the SIM functionally equivalent variant comprises or consists of the amino acid sequence of SEQ ID NO: 1 where the amino acid at position 3, 5, 6, 7, 12, 13, 14, 15, and/or 16 of SEQ ID NO: 1 is replaced by the amino acid glutamic acid (E).

In particular embodiment, the SIM functionally equivalent variant comprises or consists of the amino acid sequence of SEQ ID NO: 1 where the amino acid at position 2 and/or 18 of SEQ ID NO: 1 is replaced by the amino acid alanine (A) or serine (S).

In a particular embodiment of the polypeptide of the invention, the SIM functionally equivalent variant comprises or consists of a sequence with at least 80%, at least 85%, at least 90%, at least 95%, at least 96%, at least 97%, at least 98%, at least 99%, at least 99.9% identity with SEQ ID NO: 1 where the amino acid substitutions are selected from the above-mentioned amino acid substitutions.

It is to be understood that regardless of the mutations which the SIMs of the polypeptide of the invention may or may not have, its function of being able to interact with SUMO will be present. It will be further understood that the numbering of amino acid positions when referring to mutations is in reference to the sequence according to SEQ ID NO: 1.

The flexible linkers flanking the SIMs in the motifs present in the polypeptide of the invention may be linked to each other by a natural amino acid sequence. In a particular embodiment of the polypeptide of the invention the motif further comprises a natural amino acid sequence linker flanking the SIMs.

The term "natural linker or spacer" as used herein, refers to a natural sequence present in naturally occurring proteins that present the SUMO interacting motif described in the present invention, including PML, Daxx, UBA-2, PIAS1, XRCC4 and RNF4. In a preferred embodiment of the invention, the natural amino acid sequence linker is the linker present in RNF4 protein.

The term "PML or Promyelocytic leukemia protein" as used herein refers to a protein associated with PML nuclear bodies, also named E3 SUMO-protein ligase PML, RING finger protein 71 or Tripartite motif-containing protein 19 (TRIM19), which comprises a sequence according to the database SWISS-PROT with accession number P29590, entry version 252, sequence version 3 of the 3^{rd} of August 2022.

The term "Daxx or Death domain-associated protein 6" as used herein refers to a protein known to repress transcriptional potential of several sumoylated transcription factors, also named ETS1-associated protein 1 (EAP1) or Fas death domain-associated protein, which comprises a sequence according to the database SWISS-PROT with accession number Q9UER7, entry version 212, sequence version 2 of the 3^{rd} of August 2022.

The term "PIAS1 or Protein inhibitor of activated STAT protein 1" as used herein refers to a protein which functions as an E3-type small ubiquitin-like modifier (SUMO) ligase, also named DEAD/H box-binding protein 1, Gu-binding protein (GBP), Protein inhibitor of activated STAT protein 1 or RNA helicase II-binding protein, which comprises a sequence according to the database SWISS-PROT with accession number O75925, entry version 209, sequence version 2 of the 3^{rd} of August 2022.

The term "XRCC4 or X-ray repair cross-complementing protein 4" as used herein refers to a protein involved in DNA repair, also named DNA repair protein XRCC4, which comprises a sequence according to the database SWISS-PROT with accession number Q13426, entry version 199, sequence version 2 of the 3^{rd} of August 2022.

The term "RNF4 or RING finger protein 4" as used herein refers to a E3 ubiquitin-protein ligase which binds polysumoylated chains covalently attached to proteins and mediates 'Lys-6'-, 'Lys-11'-, 'Lys-48'- and 'Lys-63'-linked polyubiquitination of those substrates, also named E3 ubiquitin-protein ligase RNF4, RING-type E3 ubiquitin transferase RNF4 or Small nuclear ring finger protein (Protein SNURF), which comprises a sequence according to the database SWISS-PROT with accession number P78317, entry version 191, sequence version 1 of the 3^{rd} of August 2022.

The polypeptide of the invention may further comprise labels which will serve different purposes in the role of the polypeptide.

A "label" refers to a compound or composition that facilitates detection of a compound or composition with which it is specifically associated, which can include conferring a property that makes the labeled compound or composition able to bind specifically to another molecule. "Labeled" refers to a compound or composition that is specifically associated, typically by covalent bonding but non-covalent interactions can also be employed to label a compound or composition, with a label. Thus, a label may be detectable directly, i.e., the label can be a radioisotope (e.g., <3> H, <14> C, <32> P, <35> S, <125> !, <131> I) or a fluorescent or phosphorescent molecule (e.g., FITC, rhodamine, lanthanide phosphors), or indirectly, i.e., by enzymatic activity (e.g., horseradish peroxidase, beta-galactosidase, luciferase, alkaline phosphatase) or by its ability to bind to another molecule (e.g., streptavidin, biotin, an antigen, epitope, or antibody). Incorporation of a label can be achieved by a variety of means, i.e., by use of radiolabeled or biotinylated nucleotides in polymerase-mediated primer extension reactions, epitope-tagging via recombinant expression or synthetic means, or binding to an antibody. The term label can also refer to a "tag", which can bind specifically to a labeled molecule. For instance, one can use biotin as a tag and then use avidin-conjugated or streptavidin-conjugated horseradish peroxidase (HRP) to bind to the tag, and then use a chromogenic (e.g., tetramethylbenzamine) or lumigenic substrate to detect the presence of HRP. In a similar fashion, the tag can be an epitope or antigen (e.g., digoxigenin), and an enzymatically, fluorescently, or radioactively labeled antibody can be used to bind to the tag. The term "label" is also meant to refer to protein, polypeptide, compound or molecule which functions as a marker attached to the polypeptide of the invention for the purpose of identification, purification, isolation or other such purposes. These tags are useful to verify the integrity of the traps by Western blot analysis and to consider further purification steps by immunoprecipitation, when required. Tags also allow immunodetection procedures (ELISA) and the study of sub-cellular distribution by indirect immunofluorescence.

Labels can be attached directly or via spacer arms of various lengths, i.e., to reduce steric hindrance. Any of a wide variety of labeled reagents can be used for purposes of the present invention.

In a particular embodiment of the polypeptide of the invention, the polypeptide of the invention further comprises at least one label. In another particular embodiment the at least one label is selected from a group consisting of: polyhistidine, Glutathione S-transferases (GST), P and V protein of the simian virus 5 (SV5), GFP. RFP, mCherry, mVenus, YFP, CFP, FITC, rhodamine, lanthanide phosphors, horseradish peroxidase, beta-galactosidase, alkaline phosphatase, luciferase (including Nanoluc), BirA, BiolD fusions, TurbolD, BSP biotinylation target sequence, AviTag, Halo tag, SpyTag, SpyCatcher, SnoopTag, Snoopcatcher, DogTag, SnoopTagJr, Split-GFP, Split-mCherry, Split-TurboID, and any combination thereof. Split proteins for fragment complementation of any these proteins (e.g. Split-GFP, Split-mCherry, Split-TurboID, Nanoluc [Hibit, LgBit, SmBit], etc.) would also constitute labels.

In a particular embodiment, the polypeptide of the invention further comprises at least one label, at least two labels, at least three labels.

In a particular embodiment of polypeptide of the invention, the polypeptide comprises two or more labels at the N-terminus and/or C-terminus. In another particular embodiment of the polypeptide of the invention, the polypeptide comprises at least one label at its C-terminus and at least one label at its N-terminus region. In a particular embodiment of the polypeptide of the invention the at least one label is selected from a group consisting of: polyhistidine, Glutathione S-transferases (GST), P and V protein of the simian virus 5 (SV5), Maltose-binding protein (MBP), Human influenza hemagglutinin (HA) tag, c-Myc tag, FLAG tag and any combination thereof.

In another particular embodiment of the polypeptide of the invention, the polypeptide further comprises at least one label wherein the label is a polyhistidine label comprising at least 2, at least 3, at least 4, at least 5, at least 6, at least 7, at least 8, at least 9, at least 10 histidines.

In another particular embodiment of the polypeptide of the invention, the polypeptide further comprises at least one label wherein the label is a GST label comprising the sequence according to SEQ ID NO: 4.

SEQ ID NO: 4
MSPILGYWKIKGLVQPTRLLLEYLEEKYEEHLYERDEGDKWRNKKFELGLEFPNLPYYIDGDVKL
TQSMAIIRYIAKHNMLGGCPKERAEISMLEGAVLDIRYGVSRIAYSKDFETLKVDFLSKLPEMLK
MFEDRLCHKTYLNGDHVTHPFMLYDALDVVLYMDPMCLDAFPKLVCFKKRIEAIPQIDKYLKSSK
YIAWPLQGWQATFGGGDHPPKSDLEVLFGPLGS

In another particular embodiment of the polypeptide of the invention, the polypeptide further comprises at least one label wherein the label is a SV5 label comprising the sequence according to SEQ ID NO: 5.
SEQ ID NO: 5
GKPIPNPLLGLEST

In another particular embodiment of the polypeptide of the invention, the polypeptide comprises the sequence according to SEQ ID NO: 6.

SEQ ID NO: 6

MSPILGYWKIKGLVQPTRLLLEYLEEKYEEHLYERDEGDKWRNKKFELGLEFPNLPYYIDGDVKL

TQSMAIIRYIAKHNMLGGCPKERAEISMLEGAVLDIRYGVSRIAYSKDFETLKVDFLSKLPEMLK

MFEDRLCHKTYLNGDHVTHPFMLYDALDVVLYMDPMCLDAFPKLVCFKKRIEAIPQIDKYLKSSK

YIAWPLQGWQATFGGGDHPPKSDLEVLFGPLGSHHHHHHGSEGDEDDIIVVDDDDDEGEGGSGG

GSEGDEDDIIVVDDDDDEGEGGSGGGSEGDEDDIIVVDDDDDEGEGGSGGGSEGDEDDIIVV

DDDDDEGEGGSGGGSPEFPGVDLEGKPIPNPLLGLEST

In a particular embodiment of the polypeptide of the invention, the polypeptide is bound to a solid support.

The term "solid support" as used herein includes all materials on which the polypeptide of the invention may be immobilized. Natural or synthetic materials, chemically modified or otherwise, can be used as solid support, especially polysaccharides such as cellulose materials, cellulose derivatives; polymers such as polystyrene, polyacrylate, polyethylene, vinyl chloride or copolymers such as propylene and vinyl chloride polymer, vinyl chloride and vinyl acetate polymer; styrene-based copolymers; natural fibers and synthetic fibers; crosslinked polymer beads, beaded agarose, planar glass, nanoparticles and glutathione beads. In a particular embodiment of the polypeptide of the invention the polypeptide is bound to a solid support selected from a group consisting of: polystyrene, polyacrylate, polyethylene, vinyl chloride, vinyl chloride polymer, vinyl chloride and vinyl acetate polymer, crosslinked polymer beads, agarose beads, planar glass and glutathione beads. In a more particular embodiment the solid support are glutathione beads. The attachment of polypeptides or proteins to solid supports is well known in the field and several techniques are available, such as described in Camarero, J. A. 2008 (Peptide Science, vol. 90, 3, pp. 450-458), that allow for the polypeptide of the invention to be bound to a solid support.

### Polynucleotides, vectors and host cells

The polypeptide of the invention may be expressed and coded in a nucleotide sequence. Therefore, another aspect of the present invention relates to a polynucleotide encoding the polynucleotide of the invention, from the onwards the polynucleotide of the invention.

The term "nucleic acid", "nucleotide sequence", or "polynucleotide" is used interchangeably in the present invention to refer to the polymeric form of the ribonucleoside phosphate ester (adenosine, guanosine, uridine or cytidine; "RNA molecules") or deoxy ribonucleosides (deoxiadenosine, deoxyguanosine, deoxythymidine or deoxycytidine; "DNA molecules") or any phosphoester analog thereof such as phosphorothioates and thioesters, in a single stranded or double stranded form. Thus, the term includes single stranded DNA or RNA molecules. It also includes double stranded molecules formed by DNA-DNA, DNA-RNA and RNA-RNA strands. The term "nucleic acid sequence" and, in particular, the DNA or RNA molecule, refers only to the primary or secondary structure of the molecule and does not limit any particular type of tertiary structure. Thus, this term encompasses double stranded DNA, as comprised in linear or circular DNA molecules, supercoiled DNA plasmids and chromosomes. IN a particular embodiment, the nucleic acid is a DNA molecule. In another particular embodiment, it is an RNA molecule.

In a particular embodiment of the present invention the polynucleotide of the invention comprises or consists of the sequence according to SEQ ID NO: 7.

SEQ ID NO: 7
ATGTCCCCTATACTAGGTTATTGGAAAATTAAGGGCCTTGTGCAACCCACTCGACTTCTTTTGGA
ATATCTTGAAGAAAAATATGAAGAGCATTTGTATGAGCGCGATGAAGGTGATAAATGGCGAAACA
AAAAGTTTGAATTGGGTTTGGAGTTTCCCAATCTTCCTTATTATATTGATGGTGATGTTAAATTA
ACACAGTCTATGGCCATCATACGTTATATAGCTGACAAGCACAACATGTTGGGTGGTTGTCCAAA
AGAGCGTGCAGAGATTTCAATGCTTGAAGGAGCGGTTTTGGATATTAGATACGGTGTTTCGAGAA

TTGCATATAGTAAAGACTTTGAAACTCTCAAAGTTGATTTTCTTAGCAAGCTACCTGAAATGCTG
AAAATGTTCGAAGATCGTTTATGTCATAAAACATATTTAAATGGTGATCATGTAACCCATCCTGA
CTTCATGTTGTATGACGCTCTTGATGTTGTTTTATACATGGACCCAATGTGCCTGGATGCGTTCC
CAAAATTAGTTTGTTTTAAAAAACGTATTGAAGCTATCCCACAAATTGATAAGTACTTGAAATCC
AGCAAGTATATAGCATGGCCTTTGCAGGGCTGGCAAGCCACGTTTGGTGGTGGCGACCATCCTCC
AAAATCGGATCTGGAAGTTCTGTTCCAGGGGCCCCTGGGATCTCATCACCATCACCATCACGGAT
CTGAAGGAGACGAGGATGACGATATTATCGTAGTGGATGATGATGACGATGAAGGAGAAGGTGGC
TCTGGCGGTGGATCTGAAGGAGACGAGGATGACGATATTATCGTAGTGGATGATGATGACGATGA
AGGAGAAGGTGGCTCTGGCGGTGGATCTGAAGGAGACGAGGATGACGATATTATCGTAGTGGATG
ATGATGACGATGAAGGAGAAGGTGGCTCTGGCGGTGGATCTGAAGGAGACGAGGATGACGATATT
ATCGTAGTGGATGATGATGACGATGAAGGAGAAGGTGGCTCTGGCGGTGGATCCCCGGAATTCCC
CGGGGTCGACCTCGAGGGAAAGCCAATACCTAATCCACTACTTGGACTAGAATCCACATAG

The polynucleotide of the invention can be present in a vector which can be used to express the polypeptide of the invention in different host cells.

Therefore, another aspect of the present invention relates to a vector comprising the polynucleotide of the invention, from here onwards the vector of the invention.

The choice of the vector will depend on the host cell in which it is to be subsequently introduced. In a particular embodiment, the vector of the invention is an expression vector. Suitable vectors for the insertion of the polynucleotide sequence of the invention are vectors derived from prokaryotic expression vectors such as pUC 18, pUC 19, pGEX-6P-I , Bluescript and their derivatives, mp I 8, mp I9, pBR322, pMB9, ColEl, PCRI, RP4, phage and "shuttle" vectors such as pSA3 and pAT28, yeast expression vectors such as yeast 2 micron plasmid, integration plasmid, yeast episomalplasmid (YEp) vectors, similar and centromeric plasmids, expression vectors in insect cells such as vectors the pAC series and the pVL series, plant expression vectors such as vectors series plBl, pEarleyGate, pAVA, pCAMBIA, pGSA, pGWB, pMDC, pMY, pORE and similar and expression vectors in eukaryotic cells rather than based on viral vectors (adenovirus, adenovirus associated viruses and retroviruses, and particularly, lentivirus) and non-viral vectors such as pSilencer 4.1-CMV (Ambion), pcDNA3, pcDNA3.1/hyg pHCMV/Zeo, pCR3.1 , pEFI/His, pIND/GS, pRc/HCMV2, pSV40/Zeo2, PTRACE- HCMV, pUB6N5-His, pVAXI, pZeoSV2, pCI, pSVL and pKSV-10, pBPV-1, pML2d and pTDTI . By way of illustration, the vector in which said nucleic acid sequence is introduced can be a plasmid which is or is not integrated in the genome of a host cell when it is introduced in said cell. Illustrative, non-limiting examples of vectors in which the nucleotide sequence of the invention or the gene construct of the invention can be inserted include a Tet-On inducible vector for expression in eukaryote cells. The vector of the invention can be obtained by conventional methods known by persons skilled in the art (Sambrook et al., 1989). In a particular embodiment of the vector of the invention, said vector is a vector useful for transforming animal cells.

The vector of the invention can be used to transform, transfect, or infect cells which can be transformed, transfected, or infected by said vector. In this sense, another aspect of the present invention relates to a host cell comprising the polynucleotide of the invention and/or the vector of the invention and/or expressing the polypeptide of the invention. Said cells can be prokaryotic or eukaryotic. The vector of the invention can be used to transform eukaryotic cells such as yeast cells, *Saccharomyces cerevisiae,* or mammalian cells for example epithelial kidney 293 cells or U2OS cells, or HeLa cells or prokaryotic cells such as bacteria, *Escherichia coli* or *Bacillus subtilis,* for example.

### Uses and methods of the invention

All previous definitions and embodiments presented in relation with previous described aspects are equally valid for the present aspects and their embodiments.

The polypeptide of the invention finds use in the identification and/or isolation of SUMO substrates. Therefore, another aspect of the present invention relates to the use of the polypeptide of the invention for the identification and/or isolation of SUMO substrates.

The term "SUMO substrates" as used herein refers to any protein which interacts with SUMO proteins and are modified by these. Therefore, SUMO substrates are preferably SUMOylated proteins, including polySUMOylated proteins as well as monoSUMOylated proteins.

In the present invention, the term "identification" means to make the identity of a component, molecule or protein which binds directly or indirectly to the polypeptide of the invention.

The term "isolation" as used herein is intended to include the physical separation of the soluble components of interest in a solution remaining after removal of the remaining matter in said solution, and that such isolation is based upon discrimination of products on the basis of binding directly or indirectly to the polypeptide of the invention.

Another aspect of the present invention relates to an *in vitro*/*ex vivo* method for the isolation of SUMO substrates from a sample, from here onwards the isolation method of the invention, comprising the steps of:
i) incubating the polypeptide of the invention with the sample in conditions allowing for said polypeptide to interact with at least one substrate present in said sample; and
ii) recovering any SUMO substrates which is bound to the polypeptide.

The term *"in vitro"* as used herein refers to a biological processes or reactions that are conducted using components of an organism that have been isolated from their usual biological context in order to permit a more detailed or more convenient analysis than can be done with whole organisms, in an artificial environment, i.e. a laboratory.

*The term "ex vivo"* refers to a process that which takes place outside an organism. Particularly, this term refers to experimentation or measurements done in or on sample or tissue in an artificial environment outside the organism with the minimum alteration of natural conditions. Samples or tissues can be removed by any method known and used for the same purpose in the state of the art.

### • Step i)

Step i) of the isolation method of the invention relates to the process of bringing into contact the polypeptide of the invention with a sample which may contain SUMO substrates. The sample containing SUMO substrates can be any suitable source. In a particular embodiment of the isolation method of the invention the sample is a cell extract, preferably a cell extract obtained by cell lysis. The sample can be pre-treated before being used in the isolation method of the invention, in order to allow the access of its contents to the polypeptide of the invention. Therefore, in a particular embodiment of the isolation method, the sample of step i) is pretreated. Sample can be pretreated to increase the SUMOylation, decrease the proteolysis, and decrease the deubiquitination or any combination of said effects. Therefore, in a more particular embodiment of step i) of the isolation method of the invention the sample is pretreated with a stressing agent, an anti-proteolysis agent, an anti-deubiquitination agent or any combination thereafter. In a particular more particular embodiment the stressing agent is heat or arsenic trioxide (ATO). In another more particular embodiment the anti-proteolysis agent is a protease inhibitor, preferably Bortezomib and/or MG132. In yet another more particular embodiment the anti-deubiquitination agent is an ubiquitin and ubiquitin-like isopeptidases inhibitor, preferably iodoacetamide (IAA), N-Ethylmaleimide (NEM) or thiocyanic acid, C,C'-(2,6-diamino-3,5-pyridinediyl) ester (PR-619).

The expression "in conditions allowing for said polypeptide to interact with at least one substrate present in said sample" refers to the process of allowing the sample medium to interact with the polypeptide of the invention in such a way as to allow for the substrates to bind to the polypeptide. As the expert in the field understands, said conditions will invariable depend on several parameters such as the type of sample the how the polypeptide is presented to the cell. An example of such possible conditions is present in the Example section or in Da Silva-Ferrada, E. et al., 2013 (Scientific Reports volume 3, Article number: 1690) or in Xolalpa W., et al., 2016, (Rune Matthiesen (ed.), Proteostasis: Methods and Protocols, Methods in Molecular Biology, vol. 1449, pp. 161-175).

### • Step ii)

In a particular embodiment of the isolation method of the invention, step ii) is carried out by affinity column.

The isolation method of the invention can further comprise a step of washing the SUMO substrates obtained in step ii), as to remove unwanted contaminants and increase the level of purification of the SUMO substrates.

The SUMO substrates obtained in step ii) may contain besides unknown proteins, but also known proteins which are known to bind to SIMs. Therefore in a particular embodiment of the isolation method of the invention the SUMO substrates of step ii) are selected from a group consisting of: Promyelocytic leukemia protein (PML), nuclear factor of kappa light polypeptide gene enhancer in B-cells inhibitor, alpha (IκBα), tumor protein p53, Phosphatase and Tensin homolog (PTEN) or any combination thereof.

Equally, the isolation method of the invention can further comprise a step of storage of the SUMO substrates obtained in step ii). In a particular embodiment of the isolation method of the invention, the SUMO substrates obtained in step ii) are lyophilized. The term "lyophilized" or "lyophilization" as used herein refer to a process in which water is removed from a product after it is frozen and placed under a vacuum.

Yet another aspect of the present invention relates to an *in vitro*/*ex vivo* method for the identification of SUMO substrates, from here onwards the identification method of the invention, from a sample comprising the steps of:
i) incubating the polypeptide of the invention with the sample in conditions allowing for said polypeptide to interact with at least one substrate of the proteins present in said sample;
ii) recovering any SUMO substrates which is bound to the polypeptide; and
iii) identifying any of the SUMO substrates recovered in the previous step.

Step i) and step ii) of the identification method of the invention are identical to steps i) and ii) of the isolation method of the invention and the definitions and embodiments defined previously are equally valid to the present aspect of the invention.

### • Step iii)

Several techniques can be used for the identification of the SUMO substrates obtained in step ii). In a particular embodiment of the identification method of the invention, step ii) is performed by a technique selected from: edman degradation, mass spectrometry, immunoblotting, autoradiography, and any combination thereof.

In cases where the SUMO substrates have been stored previously to step iii) the SUMO substrates have to be reconstructed into an acceptable form for performing any of the techniques described previously.

A further aspect of the present invention relates to a method for the detection of SUMO substrates, from here onwards the detection method of the invention, in a sample comprising the steps of:
i) incubating the polypeptide of the invention with the sample in conditions allowing for said polypeptide to interact with at least one substrate of the proteins present in said sample;
ii) detecting the polypeptide of the previous step in the sample, wherein the detection of the polypeptide indicates the presence of any SUMO substrates.

Step i) of the detection method of the invention is identical to step i) of the isolation method of the invention and the definitions and embodiments defined previously are equally valid to the present aspect of the invention.

### • Step ii)

Step ii) of the detection method of the invention refers to the process of detecting the polypeptide of the invention through any of the known methods for detecting polypeptides in a solution/medium. The detection of the polypeptide may be facilitated by the labelling or tagging of the polypeptide of the invention, as previously described. Therefore, in a particular embodiment of the detection method of the invention, the polypeptide of the invention is labelled and the detection of step ii) is carried out by a technique selected from a group consisting of: radionuclide identification, fluorescence detection, phosphorescence detection, immunofluorescence, immunodetection, immunoprecipitation, and/or enzymatic activity detection.

### Kit of the invention

Another aspect of the present invention relates to a kit comprising the polypeptide of the invention and/or the polynucleotide of the invention and/or the vector of the invention and/or the host cell of the invention.

A "kit" is understood as a product containing the different reagents for the carrying out the methods according to the present invention. The kits of the invention may comprise a packing which allows maintaining the reagents within determined limits. Suitable materials for preparing such packings include glass, plastic (polyethylene, polypropylene, polycarbonate and the like), bottles, vials, paper, sachets and the like. The kit of the invention can additionally contain instructions for using the reagents in the method of the invention. Said instructions can be found in the form of printed material or in the form of an electronic support which can store instructions such that they can be read by a subject, such as electronic storage media (magnetic disks, tapes and the like), optical media (CD-ROM, DVD) and the like. The media can additionally or alternatively contain Internet websites providing said instructions. In a particular embodiment, said kit further comprises a solid support. In a more particular embodiment, said support is glutathione beads. In another particular embodiment, the kit of the invention further comprising all the elements necessary to carry out the methods disclosed in the present invention.

### Further aspects of the invention

The invention is further described by the following aspects:
1. A polypeptide comprising at least 2 motifs, wherein each motif comprises a small ubiquitin-related modifier (SUMO) interacting motif (SIM), wherein the SIM comprises the sequence according to SEQ ID NO: 1 or a functionally equivalent variant thereof.
2. The polypeptide according to aspect 1, wherein the polypeptide comprises between 2 and 7 motifs.
3. The polypeptide according to aspect 1 or 2, wherein the polypeptide comprises 4 motifs.
4. The polypeptide according to any one of aspects 1 to 3, wherein the SIM functionally equivalent variant comprises a sequence with at least 80% identity with SEQ ID NO: 1.
5. The polypeptide according to any one of aspects 1 to 3, wherein the SIM functionally equivalent variant has the amino acid sequence of SEQ ID NO: 1 wherein
   - the amino acid at position 8 and/or position 9 of SEQ ID NO: 1 is replaced by an amino acid selected from leucine (L), valine (V),methionine (M) or phenylalanine (F); and/or
   - the amino acid at position 10 and/or 11 of SEQ ID NO: 1 is replaced by an amino acid selected from isoleucine (I), leucine (L), methionine (M) or phenylalanine (F); and/or
   - the amino acid at position 1, 4, 17, and/or 19 of SEQ ID NO: 1 is replaced by the amino acid aspartic acid (D); and/or
   - the amino acid at position 3, 5, 6, 7, 12, 13, 14, 15, and/or 16 of SEQ ID NO: 1 is replaced by the amino acid glutamic acid (E); and/or
   - the amino acid at position 2 and/or 18 of SEQ ID NO: 1 is replaced by the amino acid alanine (A) or serine (S).
6. The polypeptide according to any one of aspects 1 to 5, wherein the SIM consists of the sequence according to SEQ ID NO: 1.
7. The polypeptide according to any one of aspects 1 to 6, wherein the motif further comprises flexible linkers flanking the SIM.
8. The polypeptide according to aspect 7 wherein the flexible linkers are glycine-serine flexible linkers and comprise a sequence consisting of GS or the sequence of SEQ ID NO: 2.
9. The polypeptide according to any one of aspects 1 to 8, wherein the motif comprises the sequence according to SEQ ID NO: 3 or a functionally equivalent variant thereof.
10. The polypeptide according to aspect 9, wherein the motif consists of the sequence according to SEQ ID NO: 3.
11. The polypeptide according to any one of aspects 1 to 10, further comprising at least one label.
12. The polypeptide according to aspect 11 comprising two or more labels at the N-terminus and/or C-terminus.
13. The polypeptide according to aspect 12, wherein the polypeptide comprises at least one label at its C-terminal and at least one label at its N-terminal.
14. The polypeptide according to any one of aspects 11 to 13, wherein the at least one label is selected from a group consisting of: polyhistidine, Glutathione S-transferases (GST), P and V protein of the simian virus 5 (SV5), GFP. RFP, mCherry, mVenus, YFP, CFP, FITC, rhodamine, lanthanide phosphors, horseradish peroxidase, beta-galactosidase, luciferase, alkaline phosphatase, luciferase, BirA, BiolD fusions, TurbolD, BSP biotinylation target sequence, AviTag, Halo tag, SpyCatchertag, and any combination thereof.
15. The polypeptide according to aspect 14, wherein the label is a polyhistidine label comprising at least 2, at least 3, at least 4, at least 5, at least 6, at least 7, at least 8, at least 9, at least 10 histidines.
16. The polypeptide according to aspect 14, wherein the label is GST comprising the sequence according to SEQ ID NO: 4.
17. The polypeptide according to aspect 14, wherein the label is SV5 comprising the sequence according to SEQ ID NO: 5.
18. The polypeptide according to any one of aspects 1 to 17, comprising the sequence according to SEQ ID NO: 6.
19. The polypeptide according to any one of aspects 1 to 18, wherein the polypeptide is bound to a solid support.
20. A polynucleotide encoding the polypeptide according to any one of aspects 1 to 19.
21. The polynucleotide according to aspect 20 comprising the sequence of SEQ ID NO: 7.
22. A vector comprising the polynucleotide according to aspect 20 or 21.
23. A host cell comprising the vector according to aspect 22.
24. The use of the polypeptide according to any one of aspects 1 to 19, for the identification and/or isolation of SUMO substrates.
25. An *in vitro*/*ex vivo* method for the isolation of SUMO substrates from a sample comprising the steps of:
   (i) incubating the polypeptide according to any one of aspects 1 to 19 with the sample in conditions allowing for said polypeptide to interact with at least one substrate present in said sample; and
   (ii) recovering any SUMO substrates which is bound to the polypeptide.
26. An *in vitro*/*ex vivo* method for the identification of SUMO substrates from a sample comprising the steps of:
   (i) incubating the polypeptide according to any one of aspects 1 to 19 with the sample in conditions allowing for said polypeptide to interact with at least one substrate of the proteins present in said sample;
   (ii) recovering any SUMO substrates which is bound to the polypeptide; and
   (iii) identifying any of the SUMO substrates recovered in the previous step.
27. An *in vitro*/*ex vivo* method for the detection of SUMO substrates in a sample comprising the steps of:
   (i) incubating the polypeptide according to any one of aspects 1 to 19 with the sample in conditions allowing for said polypeptide to interact with at least one substrate of the proteins present in said sample;
   (ii) detecting the polypeptide of the previous step in the sample, wherein the detection of the polypeptide indicates the presence of any SUMO substrates.
28. The method according to any one of aspects 25 to 27, wherein the sample is a cell extract.
29. The method according to any one of aspects 25 to 28, wherein the SUMO substrates are selected from a group consisting of: Promyelocytic leukemia protein (PML), nuclear factor of kappa light polypeptide gene enhancer in B-cells inhibitor, alpha (IκBα), tumor protein p53, Phosphatase and Tensin homolog (PTEN) or any combination thereof.
30. A kit comprising the polypeptide according to any one of aspects 1 to 19 and/or the polynucleotide according to aspect 20 or 21 and/or the vector according to aspect 24 and/or the host cell according to aspect 23.

The invention will be described by way of the following examples which are to be considered as merely illustrative and not limitative of the scope of the invention.

### EXAMPLES

### Materials and methods

### Plasmid construction

In order to directly compare new SUMO traps with previously described RNF4-based SUMO traps (Da Silva-Ferrada, et al., 2013, Sci Rep. 3:1690), the same plasmid backbone used in that study (GST-HIS-V5, a modified version of pGEX-6P-1, GE Healthcare; kind gift of MS Rodriguez) was obtained. A high-affinity SIM motif (González-Prieto, et al., 2021, Cell Rep. 34(4):108691), flanked by flexible linker sequences, was designed for cloning into GST-HIS-V5. Two oligonucleotides were synthesized (Metabion):
#4829 SIMopti.for (SEQ ID NO: 8): 
gatctGAAGGAGACGAGGATGACGATATTATCGTAGTGGATGATGATGACGATGAAGGAGAAggt
ggctctggcggtg
#4830 SIMopti.rev (SEQ ID NO: 9):

gatccaccgccagagccaccTTCTCCTTCATCGTCATCATCATCCACTACGATAATATCGTCATC
CTCGTCTCCTTCa

Oligos were reconstituted to 100µM. Annealing reactions (5µl of each oligo, 100 mM Tris-HCl (pH 7.9), 20 mM MgCl 2 , 200 mM NaCl, 2 mM DTT; 50µl final volume) were placed in a thermoblock (95°C, 5mins; then switched off and allowed to cool to room temperature). Plasmid GST-HIS-V5 (10µg) was digested with BamH1 (37°C, overnight) and then purified using mi-Gel Extraction Kit (Metabion). Ligation of the annealed oligos and the digested vector was performed using T4 DNA ligase (Thermo). Reactions were then transformed into competent *E. coli* cells (XL10-Gold; Agilent) and plated onto LB-ampicillin plates. Colonies were selected and amplified in LB for DNA minipreps. 4-5 independent clones were sent for Sanger sequencing (Stabvida) using primer pGEX5 (GGCAAGCCACGTTTGGTG - SEQ ID NO: 10). One clone with correct, in-frame insertion was selected (OptiSIM1=OS1). Successive, reiterative cloning of additional insertions of the annealed oligos into unique BamH1 sites, followed by selection and sequencing of correct clones gave rise to OS2, OS3, and OS4.

### Production of recombinant protein

Plasmids SUBE-L (original RNF4-based SUBE construct), OS1, OS2, OS3, OS4 and GST-HIS-V5 were transformed into C41-DE3-pLysS (Lucigen) and plated onto LB-ampicillin/chloramphenicol (AMP/CAM) plates. Single colonies were grown (37°C, overnight; with shaking) in 10ml LB with AMP/CAM. New flasks with 500ml LB-AMP/CAM were prepared, equilibrated to 37°C, and inoculated with 5ml of dense starter culture, and returned to shaking incubator. When logarithmic growth was reached (optical density 600nm = 0.5), flasks were cooled to 18°C. Isopropyl β-d-1-thiogalactopyranoside (IPTG) was added (0.5mM final concentration) and cultures were incubated for protein induction (18°C, overnight; with shaking). Bacteria was harvested by centrifugation (Beckman Allegra X-12R; 3250xg; 30min). Pellets were resuspended in 20ml lysis buffer [cold; 1x PBS; supplemented with 300mM NaCl, 1mM PMSF, 1x protease inhibitor cocktail (Complete EDTA-free; Roche/Merck), lysozyme (1mg/ml; Sigma)] and incubated on ice for 30mins. To reduce viscosity, lysates were sonicated on ice (5 × 30sec pulses; 10µm displacement; Soniprep 150; Sanyo). Lysates were centrifuged in polycarbonate tubes (4°C; 20,000xg; Beckman 25.50 rotor, Avanti J-20XP; 30mins). Supernatants were incubated in batch with 1ml (bed volume) of glutathione-sepharose resin (Cytiva) with gentle agitation (4°C, overnight). Lysate/resin was loaded into 10ml column (Polyprep; Biorad) and flowthrough was collected. Resin was washed extensively with wash buffer (1x PBS; supplemented with 500mM NaCl, 1mM PMSF; 0.05% Triton X-100). Columns were eluted stepwise with 1ml elution buffer (10mM reduced glutathione, 50mM Tris-HCl pH 9.5). Protein-containing fractions were identified by Bradford assay (Biorad). Fractions containing protein were pooled and dialyzed (3000 kDa mw cutoff; 3ml Pierce Slide-a-lyzer cassettes; 4°C overnight) against 2 × 2L PBS/500mM NaCl. The final dialyzed protein prep was quantified by BCA (Pierce/Thermo) and 10% sterile glycerol was added, mixed well, and aliquots were prepared for storage at -80°C.

### Cell culture and preparation of cellular extracts

293FT cells (human embryonic kidney cells, SV40T-transformed; Invitrogen) were cultured in Dulbecco's Modified Eagle Medium (DMEM), supplemented with high glucose, sodium pyruvate, L-glutamine and penicillin-streptomycin antibiotics, and containing 10% heat-inactivated fetal bovine serum (37°C, 5% CO₂). Cells were maintained in sub-confluency and mycoplasma-negative. Three 100mm plates of cells were prepared for each condition (90% confluent, 8×10⁶ cells each). Conditions were as follows: no treatment; heat shock (plates transferred to 43°C for 1hr); PR-619 treatment (10µM, 3hrs; 37°C). After treatments, plates were placed on ice. Cells were washed in cold 1xPBS, and then lysed in TUBEs lysis buffer [(20 mM phosphate buffer pH 7.5 (Na2HPO4, NaH2PO4; Sigma); 1% Igepal (Calbiochem), 2 mM EDTA (Sigma), 50 mM sodium fluoride (Sigma), 5 mM tetra-sodium pyro-phosphate (Sigma) and 10 mM β-glycerol-2-phosphate (Sigma)], supplemented with 1mM PMSF, 1x protease inhibitor cocktail (Complete EDTA-free), 50µM PR-619 (EMD-Millipore), 200nM bortezomib (MedChemExpress). Lysis buffer was added (1ml/100mm plate) and cells were collected using a cell scraper. Lysate was incubated on ice for 30mins, with gentle vortex every 10 mins. Lysates were then sonicated on ice (3 × 10sec pulses; 10µm displacement). Lysates were then cleared by centrifugation (4°C, 21,000xg; 30min). Supernatants were transferred to new tubes, quantified using BCA, and used immediately, or alternatively aliquoted and snap-frozen for storage at -80°C.

### Binding assays using high-affinity SUMO traps

For each binding reaction, 50µl glutathione-sepharose resin (50/50 slurry; 25µl settled resin bed volume; Cytiva) was aliquoted into 1.5ml tubes, and wash/equilibrated 2x with 300µl of TUBES buffer (no supplements). 25µg of each molecular trap (GST-only, OS1, OS4, or SUBE-L) was added to glutathione resin, with volume adjusted to 300µl with TUBES buffer. Traps were loaded onto resin by slow rotation on wheel (4°C; minimum 4hrs, or overnight). Tubes with resin were centrifuged (4°C, 400xg, 2mins) and supernatants removed. Bradford assay (Biorad) confirmed that molecular traps were depleted from supernatants. Loaded resin was washed 2x with 300µl TUBES buffer. 1mg of 293FT extract for each condition was prepared, adjusting the volume to 1ml with TUBEs buffer, supplemented with 1mM PMSF, 1x protease inhibitor cocktail, 50µM PR-619 200nM bortezomib. 50µl was taken as input sample, and the rest was added to loaded resins. Samples were rotated on wheel for 1hr, 4°C. After binding, tubes were centrifuged (4°C, 400xg, 2mins) and supernatants removed and saved for analysis. Resins were washed 4x with 500µl TUBES buffer (supplemented with 1mM PMSF only), and 1x with 500µl high-salt TUBES buffer (supplemented with 0.5M NaCl and 1mM PMSF). After removing all supernatant, elution of bound material was performed by adding 50µl of 2xSDS sample buffer (5X composition: 250mM Tris-HCl pH6.8, 10% SDS, 50% glycerol, 500mM DTT, 0.25% bromophenol blue) to resin, vortexing, and boiling samples (95°C, 5mins). Samples were centrifuged (21,000xg; 30sec) before SDS-PAGE (4-12% gradient gels).

### Gel electrophoresis and western blotting

Samples were centrifuged (21,000xg; 30sec) before loading 10µl and running SDS-PAGE (4-12% gradient gels). MW markers were also loaded (Bluestar Plus, Nippon Genetics). After separation, proteins were transferred to supported nitrocellulose (Cytiva) using overnight slow transfer (constant amp, 20mA per gel) in Tris-Glycine buffer with 10% ethanol. Transferred proteins were stained with Ponceau Red (Sigma) and then membranes were incubated in 1x PBT (1x PBS, 0.1% Tween-20) to remove protein stain. Membranes were blocked in 5% non-fat milk (in PBT) for 30min-1hr. Blots were incubated with primary antibody (1:1000 anti-SUMO2/3 mAb, Proteintech) for 1hr (RT). After 3x washes with PBT, secondary antibody was applied (1:5000 anti-mouse H+L, HRP-conjugated; Jackson Immunoresearch) for 30-45min. After 3x washes with PBT, blots were developed using chemiluminescence (Biorad Clarity) and imaged (iBright CL1500, Invitrogen).

### Results:

Optimized SIM traps (OS) were constructed in a sequential manner, yielding plasmids that encode 1, 2, 3, or 4 copies of the motif (OS1, OS2, OS3, OS4; Figure 1A). The OS traps were prepared in the same vector as the original SUBE-L, to allow direct comparison. In all cases, the GST-fusion proteins expressed well in bacteria, with excellent solubility and high yield (Figure 1B). As the original SUBE-L contained a total of 8 SIM motifs, OS5/6/7/8 were also prepared. OS6 and OS8 were produced and tested for binding, but did not show improved properties over OS4 (*data not shown*).

### OS4 trap improves capture of polySUMOylated proteins

Cultured cells have many SUMOylated substrates that vary throughout the cell cycle, with some studied in detail using mutagenesis, structure and functional analysis and many more using proteomic approaches to capture SUMOylated proteins and even identify the sites on which they are modified. n non-stress conditions, proteins can be monoSUMOylated, multi-monoSUMOylated and polySUMOylated. SIMs tend to have low affinity for individual SUMOs, so molecular traps of this type tend to capture/enrich monoSUMOylated substrates. However, multi-mono- and polySUMOylated substrates can theoretically bind to the multiple SIMs present in multi-module traps. Upon stress, polySUMOylation increases. Heat shock is a known cellular stress that leads to polySUMO accumulation, and also proteotoxic stress (induced by blocking deubiquitinases or the proteasome) leads to the accumulation of both polyubiquitinated and polySUMOylated proteins.

Both heat shock and inhibition of Ub/SUMO proteases were used to increase the pool of polySUMOylated proteins in treated cells. To examine whether the use of optimized SIMs could enhance the efficiency of polySUMO capture, extracts from treated cells or controls were incubated with OS1 or OS4 resin, as well as SUBE-L resin and control GST-only resin. Analysis by western blot, using SUMO2/3 antibody, showed the presence of high molecular conjugates in the inputs as well as the elutions from the molecular traps (Figure 2). In the absence of stress, low levels of SUMO conjugates are observed, with a slight enrichment in OS4 elution versus the SUBE-L. This enrichment becomes more evident when using either heat shock or PR-619-induced proteoxic stress, with OS4 clearly capturing more polySUMOylated conjugates than SUBE-L. As reported previously, some SUMO conjugates can be seen even using a single optimized SIM (OS1). While the identity of the captured proteins can only be known using proteomic analysis, the pattern of the bands seen in the OS4 versus SUBE-L is similar (especially evident with PR-619 treatment; right panel), suggesting that OS4 might not detect novel substrates, but just enrich more the same substrates. Enrichment is an important factor when dealing with proteomics, especially if analysis of scarce samples is planned.

## Claims

1. A polypeptide comprising at least 2 motifs, wherein each motif comprises a small ubiquitin-related modifier (SUMO) interacting motif (SIM), wherein the SIM comprises the sequence according to SEQ ID NO: 1 or a functionally equivalent variant thereof.

2. The polypeptide according to claim 1, wherein the polypeptide comprises between 2 and 7 motifs.

3. The polypeptide according to claim 1 or 2, wherein the SIM functionally equivalent variant comprises a sequence with at least 80% identity with SEQ ID NO: 1.

4. The polypeptide according to claim 1 or 2, wherein the SIM functionally equivalent variant has the amino acid sequence of SEQ ID NO: 1 wherein
- the amino acid at position 8 and/or position 9 of SEQ ID NO: 1 is replaced by an amino acid selected from leucine (L) or valine (V) or methionine (M) or phenylalanine (F); and/or
- the amino acid at position 10 and/or 11 of SEQ ID NO: 1 is replaced by an amino acid selected from isoleucine (I) or leucine (L) or methionine (M) or phenylalanine (F); and/or
- the amino acid at position 1, 4, 17, and/or 19 of SEQ ID NO: 1 is replaced by the amino acid aspartic acid (D); and/or
- the amino acid at position 3, 5, 6, 7, 12, 13, 14, 15, and/or 16 of SEQ ID NO: 1 is replaced by the amino acid glutamic acid (E); and/or
- the amino acid at position 2 and/or 18 of SEQ ID NO: 1 is replaced by the amino acid alanine (A) or serine (S).

5. The polypeptide according to any one of claims 1 to 4, wherein the motif further comprises flexible linkers flanking the SIM.

6. The polypeptide according to any one of claims 1 to 5, wherein the motif comprises the sequence according to SEQ ID NO: 3 or a functionally equivalent variant thereof.

7. The polypeptide according to any one of claims 1 to 6, further comprising at least one label.

8. The polypeptide according to any one of claims 1 to 7, comprising the sequence according to SEQ ID NO: 6.

9. A polynucleotide encoding the polypeptide according to any one of claims 1 to 8.

10. A vector comprising the polynucleotide according to claim 9.

11. A host cell comprising the vector according to claim 10.

12. An *in vitro*/*ex vivo* method for the isolation of SUMO substrates from a sample comprising the steps of:
- incubating the polypeptide according to any one of claims 1 to 8 with the sample in conditions allowing for said polypeptide to interact with at least one substrate present in said sample; and
- recovering any SUMO substrates which is bound to the polypeptide.

13. An *in vitro*/*ex vivo* method for the identification of SUMO substrates from a sample comprising the steps of:
- incubating the polypeptide according to any one of claims 1 to 8 with the sample in conditions allowing for said polypeptide to interact with at least one substrate of the proteins present in said sample;
- recovering any SUMO substrates which is bound to the polypeptide; and
- identifying any of the SUMO substrates recovered in the previous step.

14. An *in vitro*/*ex vivo* method for the detection of SUMO substrates in a sample comprising the steps of:
- incubating the polypeptide according to any one of claims 1 to 8 with the sample in conditions allowing for said polypeptide to interact with at least one substrate of the proteins present in said sample;
- detecting the polypeptide of the previous step in the sample, wherein the detection of the polypeptide indicates the presence of any SUMO substrates.

15. A kit comprising the polypeptide according to any one of claims 1 to 8 and/or the polynucleotide according to claim 9 and/or the vector according to claim 10 and/or the host cell according to claim 11.
